# EUROPÄISCHE PATENTSCHRIFT

(11) **EP 0 428 497 B1**
(45) Veröffentlichungstag und Bekanntmachung des Hinweises auf die Patenterteilung: **03.11.1993**
(21) Anmeldenummer: 90890277.8
(22) Anmeldetag: 11.10.1990
(51) Int. Cl.: A61F 5/04

(54) **Gelenk für eine Kniegelenkstütze**
Joint for a knee-joint brace
Articulation pour un support d'articulation de genou

(30) Priorität: 13.11.1989 AT 2590/89
(43) Veröffentlichungstag der Anmeldung: 22.05.1991
(73) Patentinhaber: BANDAGIST HEINDL GESELLSCHAFT M.B.H., A-4040 Linz (AT)
(72) Erfinder: Reisinger, Karl, A-4100 Ottensheim (AT)
(74) Vertreter: Hübscher, Heiner, Dipl.-Ing.

(56) Entgegenhaltungen:
- US-A- 4 620 532

## Beschreibung

Die Erfindungbezieht sich auf ein Gelenk für eine Kniegelenkstütze mit zwei einerseits dem Oberschenkel und anderseits dem Unterschenkel zugeordneten Schienen, die miteinander über in eine Lagerausnehmung, vorzugsweise in eine Führungskulisse, der jeweils anderen Schiene eingreifende beabstandete Gelenkzapfen gelenkig verbunden sind.

Ein derartiges Gelenk ist interner Stand der Technik.

Einer Kniegelenkstütze kommt im allgemeinen die Aufgabe zu, insbesondere nach einer Bandverletzung eine Bewegung des Kniegelenkes zumindest in einem vorgegebenen Rahmen zuzulassen, ohne eine Überlastung der Bänder befürchten zu müssen, um nicht nur einen Muskelschwund zu verhindern, sondern auch einen Muskelaufbau zu ermöglichen. Zu diesem Zweck weisen die Kniegelenkstützen beidseits des Knies zwei mit Hilfe von Bandagen am Ober- und am Unterschenkel befestigte, miteinander gelenkig verbundene Schienen auf, die aufgrund des durch sie gebildeten Gelenkes eine Zwangsführung für das physiologische Kniegelenk bilden. Zur Anpassung der Stützgelenkbewegung an die Dreh- und Gleitbewegung des physiologischen Kniegelenkes ist es bekannt, an den dem Oberschenkel zugeordneten Schienen zwei Gelenkzapfen vorzusehen, die in eine Führungskulisse der dem Unterschenkel zugehörigen Schienen eingreifen, so daß zunächst der eine Gelenkzapfen und dann nach dem Erreichen eines Anschlages der andere Gelenkzapfen die jeweilige Drehachse für das Stützgelenk bildet. Ein solches Stützgelenk mit zwei nacheinander zur Wirkung kommenden Drehachsen kann zwar die Dreh-Gleitbewegung des physiologischen Kniegelenkes nicht genau nachbilden, doch kann durch das damit verbundene Abgehen von einer genauen physiologischen Kniegelenkführung ein zunehmender Schutz des Kreuzbandes in der Streckstellungsnähe erreicht werden, wenn der Stützgelenkskondyl kleiner als der physiologische Kondyl gewählt wird. Nachteilig bei diesen bekannten Kniegelenkstützen ist allerdings, daß eine individuelle Anpassung der Stützgelenke an die jeweiligen Anforderungen hinsichtlich der zuzulassenden Bewegungsmöglichkeiten kaum erreicht werden kann und sich auf die Begrenzung des jeweils zugelassenen Schwenkwinkels beschränkt, der durch äußere, versetzbare Anschläge auf den Schienen bestimmt wird, was wiederum die Gefahr einer unerwünschten bzw. mißbräuchlichen Verstellung mit sich bringt, dazu kommt noch, daß die Bewegung der Gelenkzapfen in den Führungskulissen zu einem Verschleiß führt, der die Führungsaufgabe der Kniegelenkstütze in Frage stellen kann und die Lebensdauer der Kniegelenkstütze maßgeblich mitbestimmt.

Um bei einem gattungsfremden Stützgelenk mit einer einzigen Gelenkachse den jeweiligen Schwenkwinkel einstellen zu können, ist es bekannt (US-A-4 620 532), das Gelenk durch zwei seitliche, zur Gelenkachse koaxiale Scheiben zu umfassen, die miteinander verbunden werden und sich gegenüber einem der beiden den Schenkeln zugeordneten Schienen drehfest abstützen. Da für die andere Schiene zwischen den beiden Scheiden verstellbare Anschläge vorgesehen sind, kann diese Schiene nur innerhalb des durch diese Anschläge bestimmten Winkelbereiches genenüber der anderen Schiene ausgelenkt werden. Eine Anpassung an unterschiedliche Aufgaben der Kniegelenkführung ist somit nicht möglich.

Der Erfindung liegt die Aufgabe zugrunde, ein Gelenk für eine Kniegelenkstütze der eingangs geschilderten Art mit einfachen Mitteln so zu verbessern, daß eine weitgehende Anpassung an unterschiedliche Aufgaben der Kniegelenkführung sichergestellt werden kann. Außerdem soll der unvermeidbare Verschleiß der Stützgelenke nicht die Lebensdauer der gesamten Kniegelenkstütze bestimmen.

Die Erfindung löst die gestellte Aufgabe dadurch, daß die Lagerausnehmung für die Gelenkzapfen bzw. die Gelenkzapfen an einem an der zugehörigen Schiene auswechselbar befestigten Gelenkeinsatz vorgesehen ist bzw. sind.

Da zufolge dieser Maßnahme beispielsweise unterschiedliche Führungskulissen für die Gelenkzapfen eingesetzt werden können, kann das Gelenk auch an unterschiedliche Aufgaben der Bewegungsführung eines Kniegelenkes angepaßt werden. Zu diesem Zweck ist ja lediglich die Befestigung eines entsprechenden Gelenkeinsatzes erforderlich. Die Auswechselbarkeit der Gelenkeinsätze macht außerdem die Lebensdauer der gesamten Kniegelenkstütze von dem Verschleiß des Gelenkes unabhängig, weil ja die verschleißanfälligen Gelenkteile als Gelenkeinsätze auswechselbar an den zugehörigen Schienen befestigt sind und daher bei Bedarf erneuert werden können, ohne die Kniegelenkstütze insgesamt zerlegen zu müssen.

Die auswechselbare Befestigung der Gelenkeinsätze bietet darüber hinaus die vorteilhafte Möglichkeit, die durch das Gelenk gegebenen Endstellungen zu verlagern, ohne Einfluß auf den Bewegungsablauf zu nehmen, weil ja der Gelenkeinsatz zu diesem Zweck an der zugehörigen Schiene lediglich in unterschiedlichen Drehstellungen befestigt werden muß, was eine zusätzliche Einstellmöglichkeit der Kniegelenkstütze eröffnet. Obwohl diese Befestigungsmöglichkeit in unterschiedlichen Drehstellungen auf verschiedenen konstruktiven Wegen erreicht werden kann, ergeben sich besonders einfache Konstruktionsverhältnisse dadurch, daß der Gelenkeinsatz als Kreisscheibe mit einer Umfangsverzahnung ausgebildet ist und daß die zugehörige Schiene eine mit einer entsprechenden Innenverzahnung versehene Ausnehmung zur Aufnahme des Gelenkeinsatzes aufweist. Zu einer Drehversetzung des Gelenkeinsatzes ist es in diesem Fall lediglich notwendig, den Gelenkeinsatz aus der Aufnahmeausnehmung der Schiene in Achsrichtung der Kreisscheibe herauszunehmen, um ihn nach einer entsprechenden Verdrehung um die Kreisscheibenachse wieder in die gezahnte Aufnahmeausnehmung einzusetzen. Die Verzahnung stellt dabei nicht nur eine für die Gelenkfunktion notwendige Drehsicherung dar, sondern erlaubt auch eine Drehverstellung in vergleichsweise kleinen Drehschritten.

Die auswechselbaren Gelenkeinsätze bieten außerdem den Vorteil, daß die im Gelenkeinsatz vorgesehene Führungskulisse den gegenseitigen Drehwinkel der Schienen begrenzende Anschläge für wenigstens einen Gelenkzapfen bilden kann, so daß keine äußeren, versetzbaren Anschläge notwendig werden. Eine Änderung des zuzulassenden Drehwinkels wird ja durch das Auswechseln des Gelenkeinsatzes sichergestellt, wobei alle mit den herkömmlichen, versetzbaren Außenanschlägen zusammenhängen Nachteile entfallen.

Um trotz der auswechselbaren Befestigung der Gelenkeinsätze eine einfache, raumsparende und vor allem die Seitenstabilität der Kniegelenkstütze sichernde Konstruktion zu erhalten, kann schließlich die den Gelenkeinsatz tragende Schiene mit dem Gelenkeinsatz zwischen gabelartige Schenkel der anderen Schiene greifen. Der in eine Aufnahmeausnehmung eingesetzte Gelenkeinsatz bedarf in diesem Fall keiner gesondereten axialen Lagesicherung, weil ja die gabelartigen, den Gelenkeinsatz umgreifenden Schenkel der anderen Schiene diese Axialsicherung übernehmen.

In der Zeichnung ist der Erfindungsgegenstand beispielsweise dargestellt. Es zeigen
- Fig. 1: ein erfindungsgemäßes Gelenk für eine Kniegelenkstütze in einer schematischen, nach der Schnittlinie I-I der Fig. 2 aufgerissenen Seitenansicht,
- Fig. 2: dieses Gelenk im Schnitt nach der Linie II-II der Fig. 1 und die
- Fig. 3 und 4: zwei unterschiedliche Ausführungsbeispiele eines erfindungsgemäßen Kniegelenkes in einer der Fig. 1 entsprechenden Darstellung in einem größeren Maßstab.

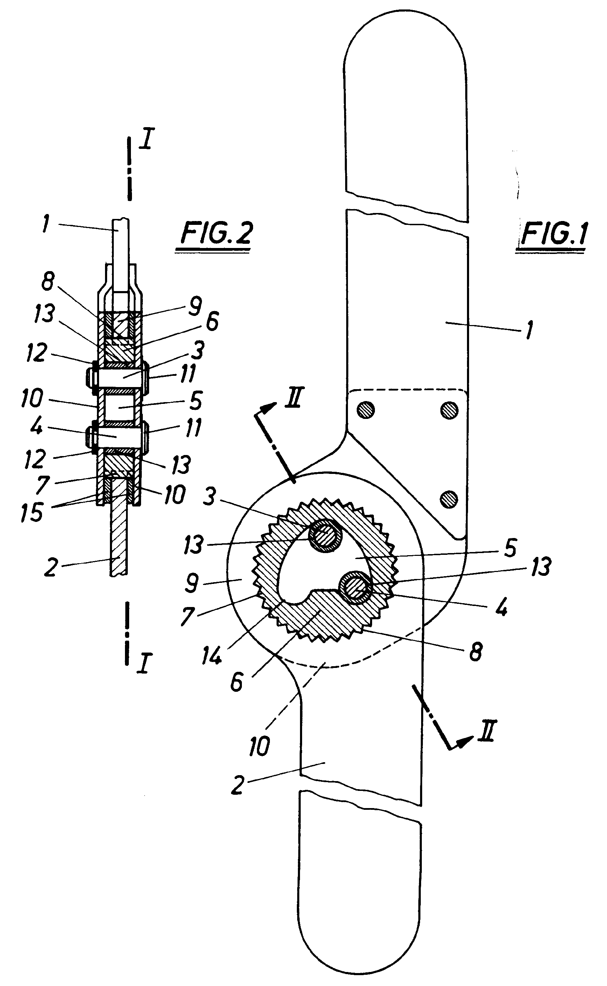

Die dargestellten Gelenke bestehen im wesentlichen aus zwei miteinander gelenkig verbundenen Schienen 1 und 2, die zusammen mit den Schienen eines gleichen Gelenkes für die gegenüberliegende Knieseite in einer Kniegelenkstütze eingebaut und mit Hilfe der Kniegelenkstütze am Bein so befestigt werden, daß die Schienen 1 mit dem Oberschenkel und die Schienen 2 mit dem Unterschenkel verbunden sind. Die gelenkige Verbindung selbst wird mit Hilfe zweier Gelenkzapfen 3 und 4 erreicht, die in eine Lagerausnehmung 5 der jeweils anderen Schiene eingreifen. Zum Unterschied zu herkömmlichen Stützgelenken dieser Art sind die Lagerausnehmungen 5 nicht in den jeweiligen Schienen selbst, sondern in einem Gelenkeinsatz 6 vorgesehen, der auswechselbar an der zugehörigen Schiene 2 befestigt ist. Zu diesem Zweck bilden der Gelenkeinsatz 6 eine Kreisscheibe mit einer Umfangsverzahnung 7 und die Schiene 2 eine Ausnehmung 8 mit einer Innenverzahnung zur Aufnahme des Gelenkeinsatzes 6. Da der den Gelenkeinsatz 6 aufnehmende Kopfteil 9 der Schiene 2 zwischen zwei diesen Kopfteil beidseitig umgreifenden, gabelartigen Schenkeln 10 der anderen Schiene 1 gehalten wird, ist eine gesonderte axiale Sicherung des Gelenkeinsatzes 6 in der Aufnahmeausnehmung 8 der Schiene 2 nicht erforderlich. Die Gelenkzapfen 3 und 4 sind in den Schenkeln 10 der Schiene 1 gehalten und durchsetzen den Gelenkeinsatz 6 in der Lagerausnehmung 5, so daß zum Auswechseln des Gelenkeinsatzes 6 lediglich die Gelenkzapfen 3 und 4 gelöst werden müssen, bevor der Schienenkopf 9 zwischen den Gabelschenkeln 10 der Schiene 1 herausgezogen werden kann. Zum leichten Lösen der an einem Ende einen Kopf 11 bildenden Gelenkzapfen 3 und 4 weisen diese im Bereich ihres anderen Endes einen Sprengring 12 für die axiale Lagesicherung auf, wie dies der Fig. 2 entnommen werden kann. Nach dem Abnehmen der Sprengringe 12 können somit die Gelenkzapfen 3, 4, die zur Reibungsverminderung und damit zur Verschleißminderung eine Rollhülse 13 durchsetzen, aus den Gabelschenkeln 10 gezogen werden. Zum Einsetzen eines neuen Gelenkeinsatzes 6 ist dieser in die gezahnte Ausnehmung 8 einzuschieben und in die durch die Schenkel 10 gebildete Gelenkgabel einzuschieben, damit die Gelenkzapfen 3 und 4 eingesetzt werden können.

Der Gelenkeinsatz 6 nach den Fig. 1 und 2 weist eine gemeinsame Lagerausnehmung 5 für beide Gelenkzapfen 3, 4 auf, wobei die eine Führungskulisse bildende Lagerausnehmung 5 die gegenseitige Drehbewegung der beiden Schienen 1 und 2 begrenzende Anschläge ergibt. In der in der Fig. 1 eingezeichneten Strecklage des Gelenkes kann die Schiene 1 um den Gelenkzapfen 4 als Drehachse verschwenkt werden, bis der Gelenkzapfen 3 den Kulissenanschlag 14 erreicht. Die weitere Drehbewegung des Gelenkes erfolgt dann um den Gelenkzapfen 3, wobei diese Drehbewegung wiederum durch die Kulissenführung begrenzt wird, wenn der Gelenkzapfen 4 an dem Kulissenast anschlägt, der die Führung des Gelenkzapfens 3 um den Gelenkzapfen 4 als Drehachse bestimmt.

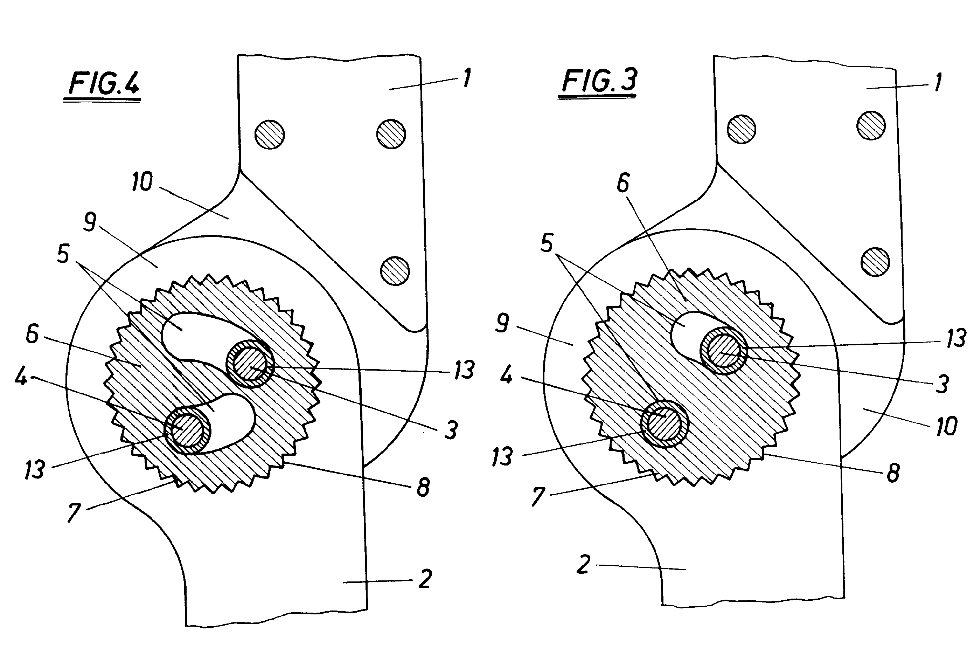

Gemäß der Fig. 3 wird ein Gelenkeinsatz 6 verwendet, der lediglich eine Drehbewegung um den Gelenkzapfen 4 als Drehachse zuläßt, weil dieser Gelenkzapfen 4 in der zugehörigen Lagerausnehmung 5 verschiebefest gehalten wird. Die zweite Lagerausnehmung 5 für den Gelenkzapfen 3 ergibt in diesem Fall lediglich eine Begrenzung des zugelassenen Drehwinkels.

Eine weitere Konstruktionsvariante ist in Fig. 4 dargestellt, in der der Gelenkeinsatz 6 je eine kreisbogenförmige Kulissenführung für die beiden Gelenkzapfen 3 und 4 bildet. Die Anordnung ist dabei so getroffen, daß die beiden Gelenkzapfen 3 und 4 jeweils in ihrer einen Endstellung die Achse für die kreisbogenartige Kulissenführung für den anderen Zapfen darstellen. Die Bogenlänge der Kulissenführung gibt dabei wiederum den jeweiligen Drehwinkel an, wobei durch den Austausch der unterschiedlichen Gelenkeinsätze 6 die jeweils gewünschte Anpassung des Stützgelenkes an die jeweiligen Bewegungsanforderungen einfach sichergestellt werden kann. Dazu kommt noch, daß durch eine Drehverstellung der Gelenkeinsätze 6 um ihre Achse die Winkellage der Endstellungen verändert werden kann, ohne den Drehwinkel zu verändern. Diese Drehverstellung erlaubt außerdem eine Berücksichtigung unterschiedlicher Winkellagen der Gelenkzapfen 3, 4, so daß auch die Gelenkeinsätze nach den Fig. 3 und 4 bei einem Gelenk nach den Fig. 1 und 2 und umgekehrt Verwendung finden können.

Um Gleitreibungen zwischen den Stirnflächen auf ein Minimum herabzusetzen, kann zwischen den gabelartigen Schenkeln 10 der Schiene 1 und dem Gelenkkopf 9 der Schiene 2 eine Gleitbeschichtung 15 vorgesehen werden, wie dies in der Fig. 2 angedeutet ist. Diese Gleitbeschichtung 15 kann sowohl dem Gelenkkopf 9 als auch den Schenkeln 10 zugeordnet werden. Soll sie auch im Bereich des Gelenkeinsatzes 6 wirksam werden, so empfiehlt sich allerdings die Beschichtung der Innenseite der Schenkel 10.

Die Erfindung ist selbstverständlich nicht auf das dargestellte Ausführungsbeispiel beschränkt. So könnten beispielsweise auch die Gelenkzapfen 3 und 4 in einem auswechselbaren Gelenkeinsatz vorgesehen sein, um eine Angleichung an eine bestimmte Kondylenform zu erreichen, die ja von dem gegenzeitigen Abstand der Gelenkzapfen abhängt. Es braucht wohl nicht erwähnt zu werden, daß in diesem Fall die Kulissenführung ebenfalls entsprechend geändert werden muß. Es könnten aber auch mehr als zwei Gelenkzapfen zum Einsatz kommen, wenn es z. B. gilt, über die Gelenkzapfen den Drehbereich zu begrenzen oder eine weiterreichende Bewegungsanpassung an das physiologische Kniegelenk sicherzustellen.

## Patentansprüche

1. Gelenk für eine Kniegelenkstütze mit zwei einerseits dem Oberschenkel und anderseits dem Unterschenkel zugeordneten Schienen (1, 2), die miteinander über in eine Lagerausnehmung (5), vorzugsweise in eine Führungskulisse, der jeweils anderen Schiene (2) eingreifende beabstandete Gelenkzapfen (3, 4) gelenkig verbunden sind, dadurch gekennzeichnet, daß die Lagerausnehmung (5) für die Gelenkzapfen (3, 4) bzw. die Gelenkzapfen (3, 4) an einem an der zugehörigen Schiene (1, 2) auswechselbar befestigten Gelenkeinsatz (6) vorgesehen ist bzw. sind.

2. Gelenk nach Anspruch 1, dadurch gekennzeichent, daß der Gelenkeinsatz (6) an der zugehörigen Schiene (2) in unterschiedlichen Drehstellungen befestigbar ist.

3. Gelenk nach den Ansprüchen 1 und 2, dadurch gekennzeichnet, daß der Gelenkeinsatz (6) als Kreisscheibe mit einer Umfangsverzahnung (7) ausgebildet ist und daß die zugehörige Schiene (2) eine mit einer entsprechenden Innenverzahnung versehene Ausnehmung (8) zur Aufnahme des Gelenkeinsatzes (6) aufweist.

4. Gelenk nach einem der Ansprüche 1 bis 3, dadurch gekennzeichnet, daß die im Gelenkeinsatz (6) vorgesehene Lagerausnehmung (5) als Führungskulisse den gegenseitigen Drehwinkel der Schiene (1, 2) begrenzende Anschläge für wenigstens einen Gelenkzapfen (3 bzw. 4) bildet.

5. Gelenk nach einem der Ansprüche 1 bis 4, dadurch gekennzeichnet, daß die den Gelenkeinsatz (6) tragende Schiene (2) mit dem Gelenkeinsatz (6) zwischen gabelartige Schenkel (10) der anderen Schiene (1) greift.

## Claims

1. A joint for a knee-joint support, comprising a rail (1) associated with the femur and a rail (2) associated with the tibia, the rails being pivotably interconnected by spaced-apart pivot pins (3, 4) engaging in a bearing recess (5), preferably in a slotted link, in the respective other rail (2), characterised in that the recess (5) for the pins (3, 4) or the pins (3, 4) is or are provided on an insert (6) interchangeably secured to the associated rail (1, 2).

2. A joint according to claim 1, characterised in that the insert (6) can be secured to the associated rail (2) in various rotary positions.

3. A joint according to claim 1 or 2, characterised in that the insert (6) is a disc with peripheral teeth (7) and the associated rail (2) has a correspondingly internally toothed recess (8) for receiving the insert (6).

4. A joint according to any of claims 1 to 3, characterised in that the bearing recess (5) in the insert (6) is a slotted link forming abutments for at least one pin (3 or 4) bounding the reciprocal angle of rotation of the rail (1, 2).

5. A joint according to any of claims 1 to 4, characterised in that the rail bearing the insert (6) engages the insert (6) of the other rail (1) between fork-like arms (10).

## Revendications

1. Articulation pour un appui à genouillère, comportant deux glissières (1, 2) associées, d'une part, à la branche supérieure et, d'autre part, à la branche inférieure, reliées ensemble de façon articulée, par l'intermédiaire de tourillons d'articulation (3, 4) espacés, s'engageant dans un évidement de tourillonnement (5), de préférence dans une coulisse de guidage, de la deuxième glissière (2),
caractérisée en ce que l'évidement de tourillonnement (5) pour les tourillons d'articulation (3, 4), respectivement les tourillons d'articulation (3, 4) est, respectivement sont, prévu(s) sur une garniture d'articulation (6) fixée de façon à pouvoir être changée, sur la glissière (1, 2) correspondante.

2. Articulation selon la revendication 1,
caractérisée en ce que la garniture d'articulation (6) peut être fixée en différentes position en rotation sur la glissière (2) correspondante.

3. Articulation selon les revendications 1 et 2,
caractérisée en ce que la garniture d'articulation (6) est réalisée sous forme de plateau circulaire, comportant une denture circonférentielle (7), et en ce que la glissière (2) correspondante présente un évidement (8), pourvu d'une denture intérieure correspondante, pour recevoir la garniture d'articulation (6).

4. Articulation selon l'une des revendications 1 à 3,
caractérisée en ce que l'évidement de tourillonnement (5) prévu dans la garniture d'articulation (6) forme des butées, limitant à titre de coulisse de guidage l'angle de rotation mutuel entre les glissières (1, 2), pour au moins un tourillon d'articulation (3 ou 4).

5. Articulation selon l'une des revendications 1 à 4,
caractérisée en ce que la glissière (2), portant la garniture d'articulation (6), s'engage avec la garniture d'articulation (6) dans l'aile fourchue (10) de l'autre glissière (1).
